# EUROPEAN PATENT APPLICATION

(11) **EP 4 201 520 A1**
(43) Date of publication of application: **28.06.2023**
(21) Application number: 21217102.9
(22) Date of filing: 22.12.2021
(51) Int. Cl.: B01J 23/63, B01J 35/02, B01J 37/02, B01J 37/08, C07C 5/333, B01J 38/12, B01J 23/96

(54) **DEHYDROGENATION CATALYST TREATMENT**

(71) Applicant: SABIC Global Technologies, B.V., 4612 PX Bergen op Zoom (NL)
(72) Inventor: Dhachapally, Naresh, 562125 Bengaluru (IN); Devassy, Biju, Maippan, 562125 Bengaluru (IN); Meleppuram, Nigit Jose, 562125 Bengaluru (IN); Nair, Vinod, Sankaran, 562125 Bengaluru (IN)
(74) Representative: Sabic INDIA Intellectual Property Group

(57) **Abstract**

A process for treating a dehydrogenation catalyst includes heating the dehydrogenation catalyst to a first temperature in the presence of a gas including oxygen for a first time period; after heating the dehydrogenation catalyst to the first temperature in the presence of the gas including oxygen, heating the dehydrogenation catalyst to a second temperature in the presence of an inert gas for a second time period; and repeating heating the dehydrogenation catalyst to the first temperature in the presence of the gas including oxygen and thereafter heating the dehydrogenation catalyst to the second temperature in the presence of the inert gas for 1 to 50 hours, or 2 to 20 hours. An example of the catalyst comprises gallium, cerium, potassium, platinum and aluminium.

## Description

### BACKGROUND

Alkane dehydrogenation is used for producing a variety of alkene products. An example of an alkene product that may be produced by alkane dehydrogenation is isobutylene, which may be used for producing methyl tert-butyl ether. Alkane dehydrogenation may be conducted in a fixed bed reactor with various types of catalysts.

### BRIEF DESCRIPTION

As described above, conventional practice provides for alkane dehydrogenation to produce a variety of alkene products. While processes and catalysts for alkane dehydrogenation exist, opportunities for improvement in alkane conversion exist and are addressed by the processes and methods of the present disclosure. Accordingly, disclosed, in various embodiments, are processes for treating a dehydrogenation catalyst and processes of dehydrogenating an alkane that have been discovered. The disclosed processes may advantageously provide for improved alkane conversion.

Disclosed herein is a process for treating a dehydrogenation catalyst including heating the dehydrogenation catalyst to a first temperature in the presence of a gas including oxygen for a first time period; after heating the dehydrogenation catalyst to the first temperature in the presence of the gas including oxygen, heating the dehydrogenation catalyst to a second temperature in the presence of an inert gas for a second time period; and repeating heating the dehydrogenation catalyst to the first temperature in the presence of the gas including oxygen and thereafter heating the dehydrogenation catalyst to the second temperature in the presence of the inert gas for 1 to 50 hours, or 2 to 20 hours.

Disclosed herein is a process of dehydrogenating an alkane including heating a dehydrogenation catalyst to a first temperature in the presence of a gas including oxygen; after heating the dehydrogenation catalyst to the first temperature in the presence of the gas including oxygen, heating the dehydrogenation catalyst to a second temperature in the presence of an inert gas; repeating heating the dehydrogenation catalyst to the first temperature in the presence of the gas including oxygen and thereafter heating the dehydrogenation catalyst to the second temperature in the presence of the inert gas for 1 to 50 hours, or 2 to 20 hours; and after the repeating, contacting the dehydrogenation catalyst with a feed including the alkane at a third temperature to produce a product stream including an alkene, wherein the third temperature is less than the first temperature, less than the second temperature, and within a range of 500 to 700 °C, or 550 °C to 675 °C.

Disclosed herein is a process of dehydrogenating an alkane including heating a dehydrogenation catalyst including gallium oxide, cerium oxide, a Group 1 metal oxide, and a Group 8-11 metal oxide on a support to a first temperature within a range of greater than 700 to 950 °C, or 725 to 900 °C, in the presence of a gas including oxygen; after heating the dehydrogenation catalyst to the first temperature in the presence of the gas including oxygen, heating the dehydrogenation catalyst to a second temperature within a range of greater than 700 to 950 °C, or 725 to 900 °C, in the presence of an inert gas; repeating heating the dehydrogenation catalyst to the first temperature in the presence of the gas including oxygen and thereafter heating the dehydrogenation catalyst to the second temperature in the presence of the inert gas for 1 to 50 hours, or 2 to 20 hours; and after the repeating, contacting the dehydrogenation catalyst with a feed including the alkane at a third temperature within a range of 500 to less than 700 °C, or 550 °C to 675 °C, to produce a product stream including an alkene.

The above described and other features are exemplified by the following figures and detailed description.

Any combination or permutation of embodiments is envisioned. Additional advantageous features, functions and applications of the disclosed catalysts, processes, and methods of the present disclosure will be apparent from the description which follows, particularly when read in conjunction with the appended figures.

### BRIEF DESCRIPTION OF THE DRAWINGS

The following figures are exemplary embodiments.

Exemplary embodiments of the present disclosure are further described with reference to the appended figures. It is to be noted that the various features, steps, and combinations of features/steps described below can be arranged and organized differently to result in embodiments which are still within the scope of the present disclosure. To assist those of ordinary skill in the art in practicing the disclosed methods and processes, reference is made to the appended figures, wherein:
FIG. 1 illustrates a flowchart of a process for treating a dehydrogenation catalyst.
FIG. 2 is a graph of isobutane conversion (percent (%)) versus number of cycles of Comparative Examples 1-3 and Example 1.
FIG. 3 is a graph of isobutylene selectivity (%) versus number of cycles of Comparative Examples 1-3 and Example 1.
FIG. 4 is a graph of isobutylene yield (%) versus number of cycles of Comparative Examples 1-3 and Example 1.
FIG. 5 is a graph of isobutane conversion (%) versus number of cycles of Comparative Example 4 and Example 2.
FIG. 6 is a graph of isobutylene selectivity (%) versus number of cycles of Comparative Example 4 and Example 2.
FIG. 7 is a graph of isobutylene yield (%) versus number of cycles of Comparative Example 4 and Example 2.

### DETAILED DESCRIPTION

The exemplary embodiments disclosed herein are illustrative of advantageous processes for treating a dehydrogenation catalyst and processes of dehydrogenating an alkane. It should be understood, however, that the disclosed embodiments are merely exemplary of the present disclosure, which may be embodied in various forms. Therefore, details disclosed herein with reference to exemplary methods and processes are not to be interpreted as limiting, but merely as the basis for teaching one skilled in the art the advantageous methods and processes of the present disclosure.

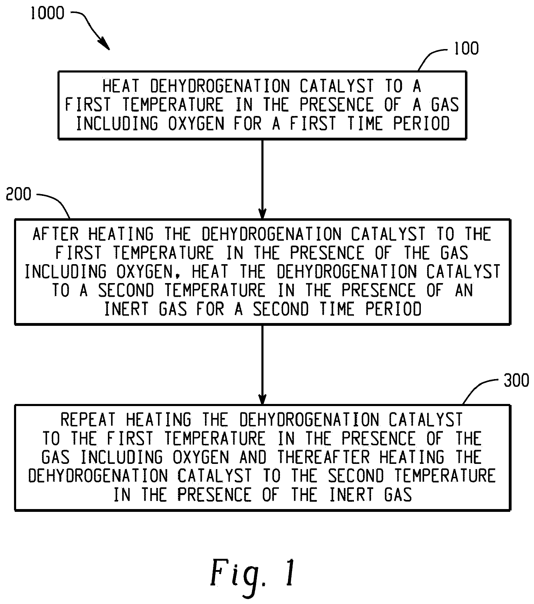

In an embodiment, with reference to FIG. 1, a process for treating a dehydrogenation catalyst (1000) includes heating the dehydrogenation catalyst to a first temperature in the presence of a gas including oxygen for a first time period (100); after heating the dehydrogenation catalyst to the first temperature in the presence of the gas including oxygen, heating the dehydrogenation catalyst to a second temperature in the presence of an inert gas for a second time period (200); and repeating heating the dehydrogenation catalyst to the first temperature in the presence of the gas including oxygen and thereafter heating the dehydrogenation catalyst to the second temperature in the presence of the inert gas (300) for 1 to 50 hours, or 2 to 20 hours. The process for treating the dehydrogenation catalyst contributes to desirable improved alkane conversion, for example, of isobutane to isobutylene.

The dehydrogenation catalyst may include gallium oxide; cerium oxide; a Group 1 metal oxide, and a Group 8-11 metal oxide; and a support. In an embodiment, the catalyst is prepared by co-extrusion.

The shape of the catalyst may include spheres, tablets, cylinders, stars, tri-lobes, quadra-lobes, pellets, pills, granules, honeycombs, and cubes. The shapes, which may be referred to as "particulates," may have any suitable size. In an embodiment, the sizes of the shapes are substantially uniform.

After forming into a desired shape, the catalyst is optionally dried to remove liquid (e.g., water). Drying the catalyst may be conducted in a desiccator, under vacuum (reduced pressure), at an elevated temperature (baking), or a combination thereof for a sufficient period of time to remove liquid.

In an embodiment, drying the catalyst involves maintaining a vacuum overnight, maintaining an elevated temperature (above about 35 °C) overnight, desiccation overnight, or a combination thereof. When employing elevated temperatures, in an embodiment, the catalyst is heated from about 35 °C to about 150 °C for a time from about 5 seconds to about 20 hours.

After drying, the catalyst may be calcined at a temperature in a range of 500 to 1,000 °C, or 600 to 975 °C or 700 to 950 °C. A temperature ramp (i.e., a heating rate) of the calcining may be in a range of 0.5 to 10 °C/minute (min), or 1 to 9 °C/min or 2 to 8 °C/min. In an embodiment, the calcination duration is in a range of 0.5 to 10 hours, or 1 to 7 hours or 1.5 to 5 hours. In an embodiment, the calcining is performed in an atmospheric environment of air, including, for example, nitrogen, oxygen, carbon dioxide, and water vapor.

The first temperature of the dehydrogenation catalyst treatment may be equal to the second temperature of the dehydrogenation catalyst treatment. Each of the first temperature and the second temperature of the dehydrogenation catalyst treatment may be within a range of 700 to 950 °C, or 725 to 900 °C.

The dehydrogenation catalyst treatment may include heating the dehydrogenation catalyst to the first temperature in the presence of the gas including oxygen for 5 to 60 minutes, or 10 to 50 minutes (i.e., a first time period). The dehydrogenation catalyst treatment may include heating the dehydrogenation catalyst to the second temperature in the presence of the inert gas for 5 to 60 minutes, or 10 to 50 minutes (i.e., a second time period).

A flow rate of the gas including oxygen may be in a range of 5 to 100 ml g⁻¹ min⁻¹, or 6 to 20 ml g⁻¹ min⁻¹. A flow rate of the inert gas may be in a range of 5 to 100 ml g⁻¹ min⁻¹, or 6 to 20 ml g⁻¹ min⁻¹.

Heating the dehydrogenation catalyst to the first temperature in the presence of the gas including oxygen may include heating at a rate of 1 to 50 degrees Celsius per minute, or 2 to 20 degrees Celsius per minute. Heating the dehydrogenation catalyst to the second temperature in the presence of the inert gas may include heating at a rate of 1 to 50 degrees Celsius per minute, or 2 to 20 degrees Celsius per minute.

The gas including oxygen may include 5 to 100 weight percent oxygen. The gas including oxygen may include air. The inert gas may include nitrogen, helium, argon, or a combination thereof.

In an embodiment, a process of dehydrogenating an alkane includes heating a dehydrogenation catalyst to a first temperature in the presence of a gas including oxygen; after heating the dehydrogenation catalyst to the first temperature in the presence of the gas including oxygen, heating the dehydrogenation catalyst to a second temperature in the presence of an inert gas; repeating heating the dehydrogenation catalyst to the first temperature in the presence of the gas including oxygen and thereafter heating the dehydrogenation catalyst to the second temperature in the presence of the inert gas for 1 to 50 hours, or 2 to 20 hours; and after the repeating, contacting the dehydrogenation catalyst with a feed including the alkane at a third temperature to produce a product stream including an alkene. The third temperature may be less than the first temperature, less than the second temperature, and within a range of 500 to 700 °C, or 550 °C to 675 °C.

In an embodiment, a process of dehydrogenating an alkane includes heating a dehydrogenation catalyst including gallium oxide, cerium oxide, a Group 1 metal oxide, and a Group 8-11 metal oxide on a support to a first temperature within a range of greater than 700 to 950 °C, or 725 to 900 °C, in the presence of a gas including oxygen; after heating the dehydrogenation catalyst to the first temperature in the presence of the gas including oxygen, heating the dehydrogenation catalyst to a second temperature within a range of greater than 700 to 950 °C, or 725 to 900 °C, in the presence of an inert gas; repeating heating the dehydrogenation catalyst to the first temperature in the presence of the gas including oxygen and thereafter heating the dehydrogenation catalyst to the second temperature in the presence of the inert gas for 1 to 50 hours, or 2 to 20 hours; and after the repeating, contacting the dehydrogenation catalyst with a feed including the alkane at a third temperature within a range of 500 to less than 700 °C, or 550 °C to 675 °C, to produce a product stream including an alkene.

In an embodiment, the alkane dehydrogenation is conducted in a fixed bed reactor. The reaction conditions may further include a reaction pressure of 0.2 to 1 bar, or 0.3 to 0.9 bar, or 0.4 to 0.8 bar. The reaction conditions may further include a gas hourly space velocity of 300 to 800 ml h⁻¹ g⁻¹, or 400 to 750 ml h⁻¹ g⁻¹, 500 to 700 ml h⁻¹ g⁻¹.

This disclosure is further illustrated by the following examples, which are non-limiting.

### EXAMPLES

### Catalyst Preparation

Catalyst was prepared by co-extrusion using a peptizing agent. Gallium nitrate hexahydrate (Ga(NO₃)₃·6H₂O, Sigma-Aldrich), tetraamineplatinum nitrate (Pt(NH₃)₄(NO₃)₂, Sigma-Aldrich), cerium nitrate hexahydrate (Ce(NO₃)₃·6H₂O, Sigma-Aldrich), potassium nitrate (KNO₃, Sigma-Aldrich), and boehmite (G-250, Chika Pvt. Ltd.) were used as precursors of gallium, platinum, cerium, potassium, and aluminum, respectively. Prior to preparing the catalyst, metal salt-containing dilute nitric acid solution was prepared. To 36 milliliters (ml) of 70 weight percent (wt. %) nitric acid, water was added to make 500 ml of solution. To prepare metal salt-containing nitric acid solution, about 8.253 grams (g) of gallium nitrate hexahydrate, 1 ml of 1.5 % tetraamineplatinum nitrate solution (1.5 g/100 ml solution), 2.32 g of cerium nitrate hexahydrate, and 1.466 g potassium nitrate were dissolved in 60 ml of the prepared nitric acid solution, and stirred to form a clear solution. The formed solution was used as peptizing agent for the preparation of the catalyst. To boehmite powder (94.6 g) (the weight loss considered is about 25%) metal salt-containing nitric acid solution was added dropwise and the mixture was mixed for about 30 minutes. The obtained dough was then extruded using a lab extruder (Sunsai), using a die having circular openings with 3.5 millimeter (mm) diameter. The prepared wet extrudates were dried at 120 °C for 16 hours in air in an oven. The dried sample was then calcined at 600 °C for 30 minutes (min) with a heating rate of 5 degrees Celsius per minute (°C/min) in the presence of air (flow rate = 8 milliliters per gram per minute (ml g⁻¹ min⁻¹)) in a horizontal flow rotating tubular reactor. After calcination, the catalyst was cooled in the presence of air and was stored in a container. The obtained extrudate catalyst size was about 2.8 mm diameter and about 6 - 8 mm length. The final calculated composition of the catalyst corresponded to 2.7 wt. % Ga₂O₃, 100 parts per million by weight (ppmw) Pt (116.4 ppmw PtO₂), 1.17 wt. % Ce₂O₃, and 0.9 wt. % K₂O, with the remainder comprising Al₂O₃. The obtained extrudate catalyst was crushed and sieved into 0.4 - 0.5 mm in size.

### Comparative Example 1

The sieved catalyst was loaded into a tubular fixed-bed quartz reactor. The details of the catalyst loading and the reactor were as follows: catalyst weight = 4.0 g, catalyst shape: particles (0.4 - 0.5 mm), reactor inner diameter (ID) = 16 mm, reactor outer diameter (OD) = 19 mm, and thermowell OD = 2.0 mm. A thermowell was placed in the middle of the catalyst bed. The catalyst was loaded into the reactor under the isothermal zone. The quartz chips with size of 1 to 1.4 mm were loaded above and below the catalyst bed. The calcined catalyst was treated by heating at 800 °C for 20 hours in air (10 ml g⁻¹ min⁻¹).

After treating, the catalyst was used for the dehydrogenation reaction. Isobutane (99.9 vol. %) was used as the feed. A nitrogen purge separated the dehydrogenation and regeneration/oxidation steps. The total isobutane feed flow in the dehydrogenation step corresponded to a gas hourly space velocity (GHSV) of 600 milliliters per hour per gram (ml h⁻¹ g⁻¹). The reactor outlet gases were analyzed by an online gas chromatograph (GC) (Agilent 6890, Agilent Scientific Instruments, USA) equipped with a flame ionization detector for hydrocarbon analysis and a thermal conductivity detector for hydrogen analysis. The reactant and products flow rates were measured using a Ritter type wet gas flow meter. The reactor was operated at about 0.5 bar partial pressure of isobutane by diluting isobutane gas with N₂ gas in a 1:1 volume ratio. The reaction was carried out in a cyclic mode with the following steps:
1. Oxidation in air at 650 °C for 10 min;
2. Cooling with nitrogen from 650 to 575 °C and hold for 20 min at 575 °C for temperature stabilization;
3. Start isobutane feed flow mixed with nitrogen (1:1) for dehydrogenation at 575 °C for 10 min;
4. GC analysis at the 9^{th} minute from the start of the isobutane feed; and
5. Increase the temperature in nitrogen from 575 to 650 °C and hold for 5 min at 650 °C for temperature stabilization.
Steps 1-5 were repeated for 100 cycles.

### Comparative Example 2

The sieved catalyst was loaded into a tubular fixed-bed quartz reactor. The details of the catalyst loading and the reactor were as follows: catalyst weight = 4.0 g, catalyst shape: particles (0.4 - 0.5 mm), reactor ID = 16 mm, reactor OD = 19 mm, and thermowell OD = 2.0 mm. A thermowell was placed in the middle of the catalyst bed. The catalyst was loaded into the reactor under the isothermal zone. The quartz chips with size of 1 to 1.4 mm were loaded above and below the catalyst bed. The calcined catalyst was treated by heating at 800 °C for 10 hours in air (10 ml g⁻¹ min⁻¹).

After treating, the catalyst was used for the dehydrogenation reaction. Isobutane (99.9 vol. %) was used as the feed. A nitrogen purge separated the dehydrogenation and regeneration/oxidation steps. The total isobutane feed flow in the dehydrogenation step corresponded to a GHSV of 600 ml h⁻¹ g⁻¹. The reactor outlet gases were analyzed by an online gas chromatograph (Agilent 6890, Agilent Scientific Instruments, USA) equipped with a flame ionization detector for hydrocarbon analysis and a thermal conductivity detector for hydrogen analysis. The reactant and products flow rates were measured using a Ritter type wet gas flow meter. The reactor was operated at about 0.5 bar partial pressure of isobutane by diluting isobutane gas with N₂ gas in a 1:1 volume ratio. The reaction was carried out in a cyclic mode with the following steps:
1. Oxidation in air at 650 °C for 10 min;
2. Cooling with nitrogen from 650 to 575 °C and hold for 20 min at 575 °C for temperature stabilization;
3. Start isobutane feed flow mixed with nitrogen (1:1) for dehydrogenation at 575 °C for 10 min;
4. GC analysis at the 9^{th} minute from the start of the isobutane feed; and
5. Increase the temperature in nitrogen from 575 to 650 °C and hold for 5 min at 650 °C for temperature stabilization.
Steps 1-5 were repeated for 150 cycles.

### Comparative Example 3

The sieved catalyst was loaded into a tubular fixed-bed quartz reactor. The details of the catalyst loading and the reactor were as follows: catalyst weight = 4.0 g, catalyst shape: particles (0.4 - 0.5 mm), reactor ID = 16 mm, reactor OD = 19 mm, and thermowell OD = 2.0 mm. A thermowell was placed in the middle of the catalyst bed. The catalyst was loaded into the reactor under the isothermal zone. The quartz chips with size of 1 to 1.4 mm were loaded above and below the catalyst bed. The calcined catalyst was treated by heating at 800 °C for 10 hours in nitrogen (10 ml g⁻¹ min⁻¹).

After treating, the catalyst was used for the dehydrogenation reaction. Isobutane (99.9 vol. %) was used as the feed. A nitrogen purge separated the dehydrogenation and regeneration/oxidation steps. The total isobutane feed flow in the dehydrogenation step corresponded to a GHSV of 600 ml h⁻¹ g⁻¹. The reactor outlet gases were analyzed by an online gas chromatograph (Agilent 6890, Agilent Scientific Instruments, USA) equipped with a flame ionization detector for hydrocarbon analysis and a thermal conductivity detector for hydrogen analysis. The reactant and products flow rates were measured using a Ritter type wet gas flow meter. The reactor was operated at about 0.5 bar partial pressure by diluting isobutane gas with N₂ gas in a 1:1 volume ratio. The reaction was carried out in a cyclic mode with the following steps:
1. Oxidation in air at 650 °C for 10 min;
2. Cooling with nitrogen from 650 to 575 °C and hold for 20 min at 575 °C for temperature stabilization;
3. Start isobutane feed flow mixed with nitrogen (1:1) for dehydrogenation at 575 °C for 10 min;
4. GC analysis at the 9^{th} minute from the start of the isobutane feed; and
5. Increase the temperature in nitrogen from 575 to 650 °C and hold for 5 min at 650 °C for temperature stabilization.
Steps 1-5 were repeated for 150 cycles.

### Comparative Example 4

The sieved catalyst was loaded into a tubular fixed-bed quartz reactor. The details of the catalyst loading and the reactor were as follows: catalyst weight = 4.0 g, catalyst shape: particles (0.4 - 0.5 mm), reactor ID = 16 mm, reactor OD =19 mm, and thermowell OD = 2.0 mm. A thermowell was placed in the middle of the catalyst bed. The catalyst was loaded into the reactor under the isothermal zone. The quartz chips with size of 1 to 1.4 mm were loaded above and below the catalyst bed. The calcined catalyst was treated by heating at 800 °C for 2 hours in air (10 ml g⁻¹ min⁻¹).

After treating, the catalyst was used for the dehydrogenation reaction. Isobutane (99.9 vol. %) was used as the feed. A nitrogen purge separated the dehydrogenation and regeneration/oxidation steps. The total isobutane feed flow in the dehydrogenation step corresponded to a GHSV of 600 ml h⁻¹ g⁻¹. The reactor outlet gases were analyzed by an online gas chromatograph (Agilent 6890, Agilent Scientific Instruments, USA) equipped with a flame ionization detector for hydrocarbon analysis and a thermal conductivity detector for hydrogen analysis. The reactant and products flow rates were measured using a Ritter type wet gas flow meter. The reactor was operated at about 0.5 bar partial pressure of isobutane by diluting isobutane gas with N₂ gas in a 1:1 volume ratio. The reaction was carried out in a cyclic mode with the following steps:
1. Oxidation in air at 650 °C for 10 min;
2. Cooling with nitrogen from 650 to 575 °C and hold for 20 min at 575 °C for temperature stabilization;
3. Start isobutane feed flow mixed with nitrogen (1:1) for dehydrogenation at 575 °C for 10 min;
4. GC analysis at the 9^{th} minute from the start of the isobutane feed; and
5. Increase the temperature in nitrogen from 575 to 650 °C and hold for 5 min at 650 °C for temperature stabilization.
Steps 1-5 were repeated for 100 cycles.

### Example 1

The sieved catalyst was loaded into a tubular fixed-bed quartz reactor. The details of the catalyst loading and the reactor were as follows: catalyst weight = 4.0 g, catalyst shape: particles (0.4 - 0.5 mm), reactor ID = 16 mm, reactor OD = 19 mm, and thermowell OD = 2.0 mm. A thermowell was placed in the middle of the catalyst bed. The catalyst was loaded into the reactor under the isothermal zone. The quartz chips with size of 1 to 1.4 mm were loaded above and below the catalyst bed. The calcined catalyst was treated with the following steps:
1. Catalyst heated at 800 °C for 20 min in air (10 ml g⁻¹ min⁻¹); and
2. Catalyst heated at 800 °C for 20 min in nitrogen (10 ml g⁻¹ min⁻¹).
Steps 1 and 2 were repeated in a cyclic mode for 20 hours.

After treating, the catalyst was used for the dehydrogenation reaction. Isobutane (99.9 vol. %) was used as the feed. A nitrogen purge separated the dehydrogenation and regeneration/oxidation steps. The total isobutane feed flow in the dehydrogenation step corresponded to a GHSV of 600 ml h⁻¹ g⁻¹. The reactor outlet gases were analyzed by an online gas chromatograph (Agilent 6890, Agilent Scientific Instruments, USA) equipped with a flame ionization detector for hydrocarbon analysis and a thermal conductivity detector for hydrogen analysis. The reactant and products flow rates were measured using a Ritter type wet gas flow meter. The reactor was operated at about 0.5 bar partial pressure by diluting isobutane gas with N₂ gas in a 1:1 volume ratio. The reaction was carried out in a cyclic mode with the following steps:
1. Oxidation in air at 650 °C for 10 min;
2. Cooling with nitrogen from 650 to 575 °C and hold for 20 min at 575 °C for temperature stabilization;
3. Start isobutane feed flow mixed with nitrogen (1:1) for dehydrogenation at 575 °C for 10 min;
4. GC analysis at the 9^{th} minute from the start of the isobutane feed; and
5. Increase the temperature in nitrogen from 575 to 650 °C and hold for 5 min at 650 °C for temperature stabilization.
Steps 1-5 were repeated for 150 cycles.

### Example 2

The sieved catalyst was loaded into a tubular fixed-bed quartz reactor. The details of the catalyst loading and the reactor were as follows: catalyst weight = 4.0 g, catalyst shape: particles (0.4 - 0.5 mm), reactor ID = 16 mm, reactor OD = 19 mm, and thermowell OD = 2.0 mm. A thermowell was placed in the middle of the catalyst bed. The catalyst was loaded into the reactor under the isothermal zone. The quartz chips with size of 1 to 1.4 mm were loaded above and below the catalyst bed. The calcined catalyst was treated with the following steps:
1. Catalyst heated at 800 °C for 20 min in air (10 ml g⁻¹ min⁻¹); and
2. Catalyst heated at 800 °C for 20 min in nitrogen (10 ml g⁻¹ min⁻¹).
Steps 1 and 2 were repeated in a cyclic mode for 2 hours.

After treating, the catalyst was used for the dehydrogenation reaction. Isobutane (99.9 vol. %) was used as the feed. A nitrogen purge separated the dehydrogenation and regeneration/oxidation steps. The total isobutane feed flow in the dehydrogenation step corresponded to a GHSV of 600 ml h⁻¹ g⁻¹. The reactor outlet gases were analyzed by an online gas chromatograph (Agilent 6890, Agilent Scientific Instruments, USA) equipped with a flame ionization detector for hydrocarbon analysis and a thermal conductivity detector for hydrogen analysis. The reactant and products flow rates were measured using a Ritter type wet gas flow meter. The reactor was operated at about 0.5 bar partial pressure by diluting isobutane gas with N₂ gas in a 1:1 volume ratio. The reaction was carried out in a cyclic mode with the following steps:
1. Oxidation in air at 650 °C for 10 min;
2. Cooling with nitrogen from 650 to 575 °C and hold for 20 min at 575 °C for temperature stabilization;
3. Start isobutane feed flow mixed with nitrogen (1:1) for dehydrogenation at 575 °C for 10 min;
4. GC analysis at the 9^{th} minute from the start of the isobutane feed; and
5. Increase the temperature in nitrogen from 575 to 650 °C and hold for 5 min at 650 °C for temperature stabilization.
Steps 1-5 were repeated for 100 cycles.

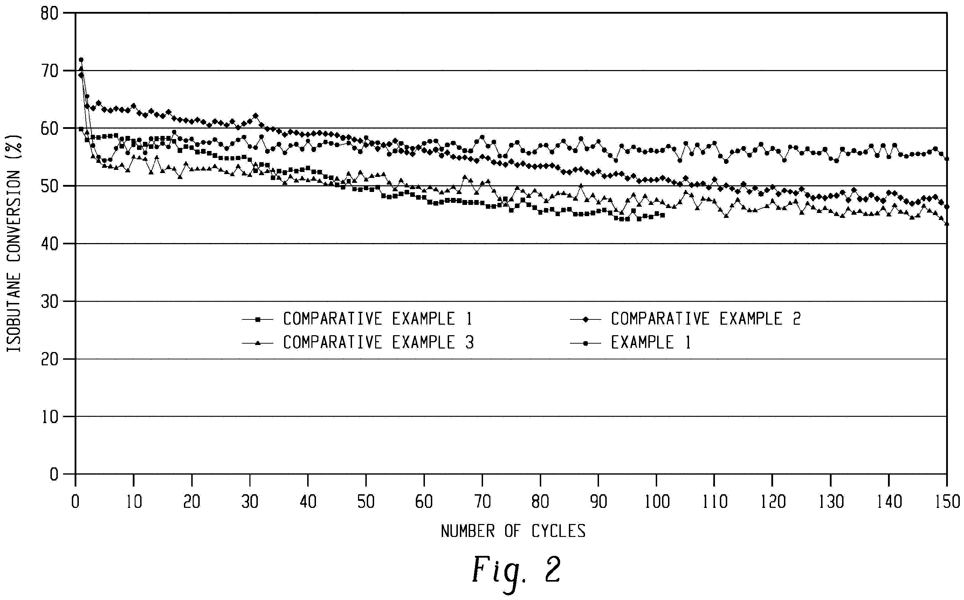

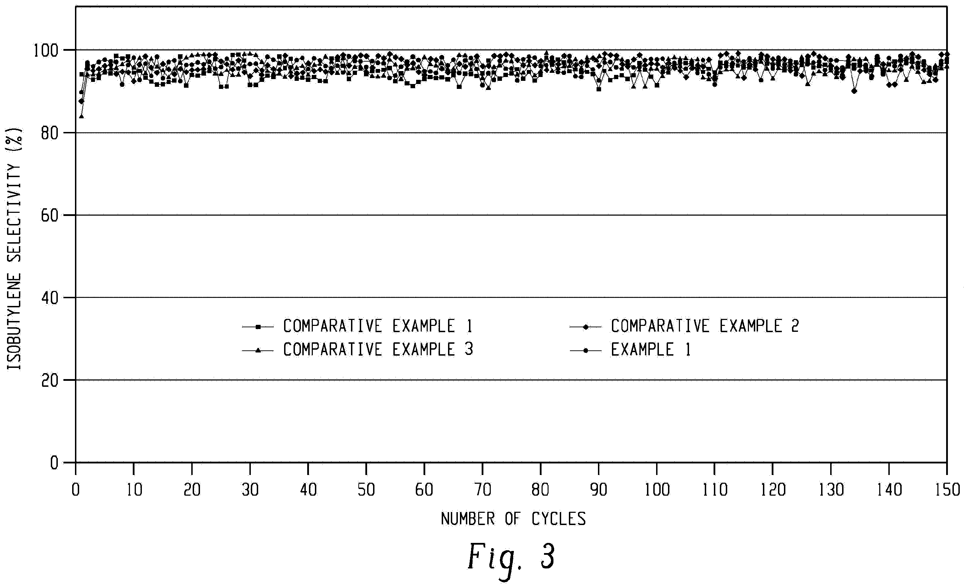

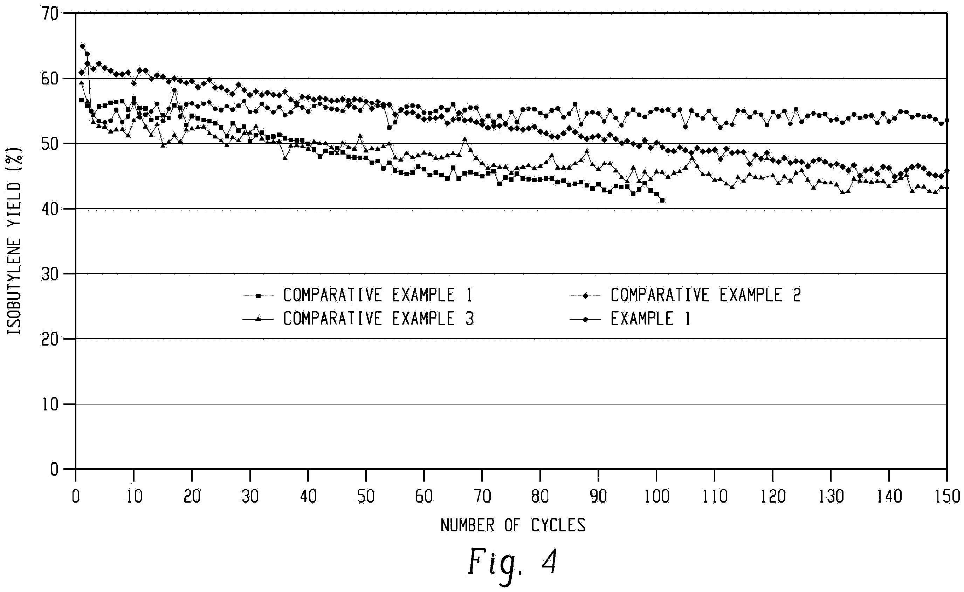

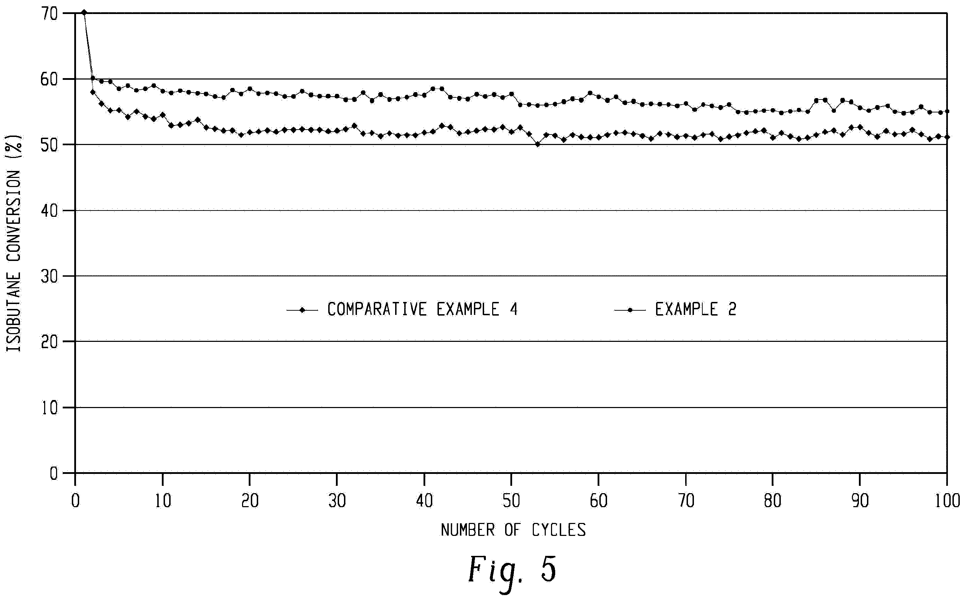

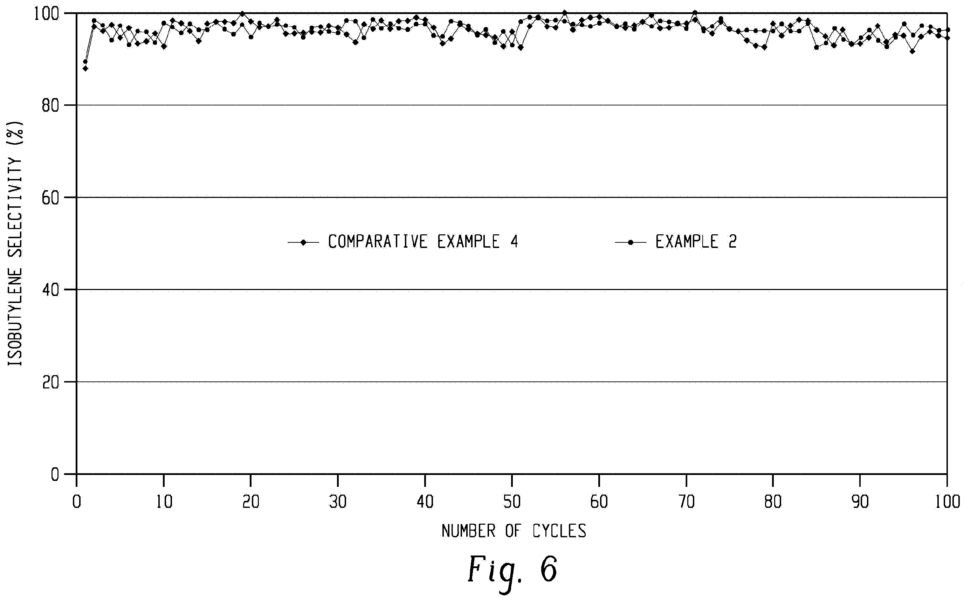

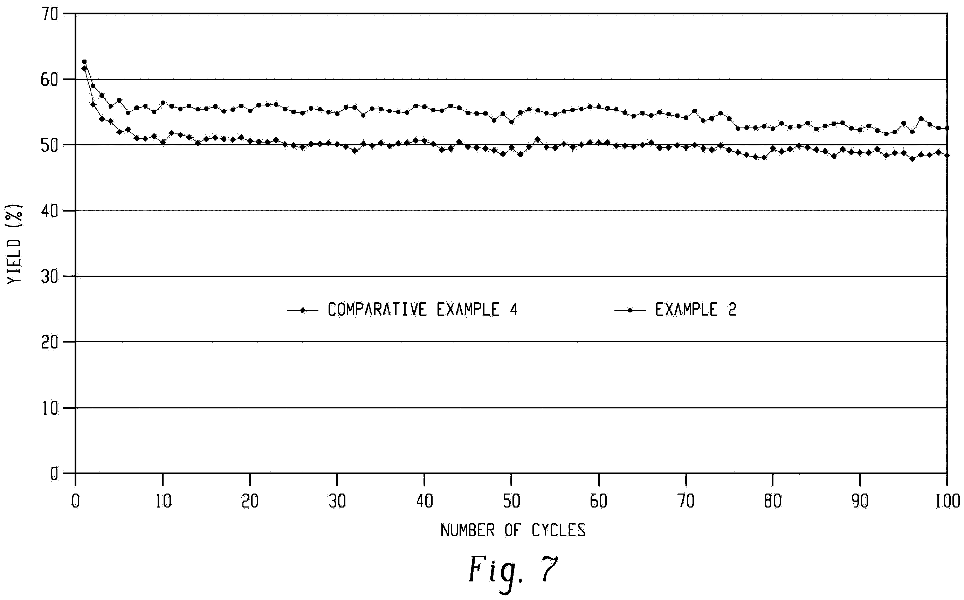

The performance of Comparative Examples 1-3 and Example 1 is shown in FIGS. 2-4 and the performance of Comparative Example 4 and Example 2 is shown in FIGS. 5-7. The results show that the activity increased for the catalyst treated in the presence of air and nitrogen in a cyclic mode.

This disclosure further encompasses the following aspects.
Aspect 1. A process for treating a dehydrogenation catalyst, the process comprising heating the dehydrogenation catalyst to a first temperature in the presence of a gas comprising oxygen for a first time period; after heating the dehydrogenation catalyst to the first temperature in the presence of the gas comprising oxygen, heating the dehydrogenation catalyst to a second temperature in the presence of an inert gas for a second time period; and repeating heating the dehydrogenation catalyst to the first temperature in the presence of the gas comprising oxygen and thereafter heating the dehydrogenation catalyst to the second temperature in the presence of the inert gas for 1 to 50 hours, or 2 to 20 hours.
Aspect 2. The process of Aspect 1, wherein the first temperature is equal to the second temperature.
Aspect 3. The process of Aspect 1 or 2, wherein the first time period is in a range of 5 to 60 minutes, or 10 to 50 minutes.
Aspect 4. The process of any of the preceding aspects, wherein the second time period is in a range of 5 to 60 minutes, or 10 to 50 minutes.
Aspect 5. The process of any of the preceding aspects, wherein a flow rate of the gas comprising oxygen is in a range of 5 to 100 ml g-1 min-1, or 6 to 20 ml g-1 min-1.
Aspect 6. The process of any of the preceding aspects, wherein a flow rate of the inert gas is in a range of 5 to 100 ml g-1 min-1, or 6 to 20 ml g-1 min-1.
Aspect 7. The process of any of the preceding aspects, wherein heating the dehydrogenation catalyst to the first temperature in the presence of the gas comprising oxygen comprises heating at a rate of 1 to 50 degrees Celsius per minute, or 2 to 20 degrees Celsius per minute.
Aspect 8. The process of any of the preceding aspects, wherein heating the dehydrogenation catalyst to the second temperature in the presence of the inert gas comprises heating at a rate of 1 to 50 degrees Celsius per minute, or 2 to 20 degrees Celsius per minute.
Aspect 9. The process of any of the preceding aspects, wherein each of the first temperature and the second temperature is within a range of 700 to 950 °C, or 725 to 900 °C.
Aspect 10. The process of any of the preceding aspects, wherein the gas comprising oxygen comprises 5 to 100 weight percent oxygen.
Aspect 11. The process of any of the preceding aspects, wherein the gas comprising oxygen comprises air.
Aspect 12. The process of any of the preceding aspects, wherein the inert gas comprises nitrogen, helium, argon, or a combination thereof.
Aspect 13. The process of any of the preceding aspects, wherein the dehydrogenation catalyst comprises: gallium oxide; cerium oxide; a Group 1 metal oxide, and a Group 8-11 metal oxide; and a support.
Aspect 14. A process of dehydrogenating an alkane, the process comprising: heating a dehydrogenation catalyst to a first temperature in the presence of a gas comprising oxygen; after heating the dehydrogenation catalyst to the first temperature in the presence of the gas comprising oxygen, heating the dehydrogenation catalyst to a second temperature in the presence of an inert gas; repeating heating the dehydrogenation catalyst to the first temperature in the presence of the gas comprising oxygen and thereafter heating the dehydrogenation catalyst to the second temperature in the presence of the inert gas for 1 to 50 hours, or 2 to 20 hours; and after the repeating, contacting the dehydrogenation catalyst with a feed comprising the alkane at a third temperature to produce a product stream comprising an alkene, wherein the third temperature is less than the first temperature, less than the second temperature, and within a range of 500 to 700 °C, or 550 °C to 675 °C.
Aspect 15. A process of dehydrogenating an alkane, the process comprising: heating a dehydrogenation catalyst comprising gallium oxide, cerium oxide, a Group 1 metal oxide, and a Group 8-11 metal oxide on a support to a first temperature within a range of greater than 700 to 950 °C, or 725 to 900 °C, in the presence of a gas comprising oxygen; after heating the dehydrogenation catalyst to the first temperature in the presence of the gas comprising oxygen, heating the dehydrogenation catalyst to a second temperature within a range of greater than 700 to 950 °C, or 725 to 900 °C, in the presence of an inert gas; repeating heating the dehydrogenation catalyst to the first temperature in the presence of the gas comprising oxygen and thereafter heating the dehydrogenation catalyst to the second temperature in the presence of the inert gas for 1 to 50 hours, or 2 to 20 hours; and after the repeating, contacting the dehydrogenation catalyst with a feed comprising the alkane at a third temperature within a range of 500 to less than 700 °C, or 550 °C to 675 °C, to produce a product stream comprising an alkene.

The compositions, methods, and articles can alternatively comprise, consist of, or consist essentially of, any appropriate materials, steps, or components herein disclosed. The compositions, methods, and articles can additionally, or alternatively, be formulated so as to be devoid, or substantially free, of any materials (or species), steps, or components, that are otherwise not necessary to the achievement of the function or objectives of the compositions, methods, and articles.

All ranges disclosed herein are inclusive of the endpoints, and the endpoints are independently combinable with each other (e.g., ranges of "up to 25 wt%, or, more specifically, 5 wt% to 20 wt%", is inclusive of the endpoints and all intermediate values of the ranges of "5 wt% to 25 wt%," etc.). "Combinations" is inclusive of blends, mixtures, alloys, reaction products, and the like. The terms "a" and "an" and "the" do not denote a limitation of quantity and are to be construed to cover both the singular and the plural, unless otherwise indicated herein or clearly contradicted by context. "Or" means "and/or" unless clearly stated otherwise. Reference throughout the specification to "some embodiments", "an embodiment", and so forth, means that a particular element described in connection with the embodiment is included in at least one embodiment described herein, and may or may not be present in other embodiments. In addition, it is to be understood that the described elements may be combined in any suitable manner in the various embodiments. A "combination thereof' is open and includes any combination comprising at least one of the listed components or properties optionally together with a like or equivalent component or property not listed.

Unless specified to the contrary herein, all test standards are the most recent standard in effect as of the filing date of this application, or, if priority is claimed, the filing date of the earliest priority application in which the test standard appears.

Unless defined otherwise, technical and scientific terms used herein have the same meaning as is commonly understood by one of skill in the art to which this application belongs. All cited patents, patent applications, and other references are incorporated herein by reference in their entirety. However, if a term in the present application contradicts or conflicts with a term in the incorporated reference, the term from the present application takes precedence over the conflicting term from the incorporated reference.

While particular embodiments have been described, alternatives, modifications, variations, improvements, and substantial equivalents that are or may be presently unforeseen may arise to applicants or others skilled in the art. Accordingly, the appended claims as filed and as they may be amended are intended to embrace all such alternatives, modifications variations, improvements, and substantial equivalents.

Although the processes and methods of the present disclosure have been described with reference to exemplary embodiments thereof, the present disclosure is not limited to such exemplary embodiments and/or implementations. Rather, the processes and methods of the present disclosure are susceptible to many implementations and applications, as will be readily apparent to persons skilled in the art from the disclosure hereof. The present disclosure expressly encompasses such modifications, enhancements and/or variations of the disclosed embodiments. Since many changes could be made in the above construction and many widely different embodiments of this disclosure could be made without departing from the scope thereof, it is intended that all matter contained in the drawings and specification shall be interpreted as illustrative and not in a limiting sense. Additional modifications, changes, and substitutions are intended in the foregoing disclosure. Accordingly, it is appropriate that the appended claims be construed broadly and in a manner consistent with the scope of the disclosure.

## Claims

1. A process for treating a dehydrogenation catalyst, the process comprising:
heating the dehydrogenation catalyst to a first temperature in the presence of a gas comprising oxygen for a first time period;
after heating the dehydrogenation catalyst to the first temperature in the presence of the gas comprising oxygen, heating the dehydrogenation catalyst to a second temperature in the presence of an inert gas for a second time period; and
repeating heating the dehydrogenation catalyst to the first temperature in the presence of the gas comprising oxygen and thereafter heating the dehydrogenation catalyst to the second temperature in the presence of the inert gas for 1 to 50 hours, or 2 to 20 hours.

2. The process of Claim 1, wherein the first temperature is equal to the second temperature.

3. The process of Claim 1 or 2, wherein the first time period is in a range of 5 to 60 minutes, or 10 to 50 minutes.

4. The process of any of the preceding claims, wherein the second time period is in a range of 5 to 60 minutes, or 10 to 50 minutes.

5. The process of any of the preceding claims, wherein a flow rate of the gas comprising oxygen is in a range of 5 to 100 ml g⁻¹ min⁻¹, or 6 to 20 ml g⁻¹ min⁻¹.

6. The process of any of the preceding claims, wherein a flow rate of the inert gas is in a range of 5 to 100 ml g⁻¹ min⁻¹, or 6 to 20 ml g⁻¹ min⁻¹.

7. The process of any of the preceding claims, wherein heating the dehydrogenation catalyst to the first temperature in the presence of the gas comprising oxygen comprises heating at a rate of 1 to 50 degrees Celsius per minute, or 2 to 20 degrees Celsius per minute.

8. The process of any of the preceding claims, wherein heating the dehydrogenation catalyst to the second temperature in the presence of the inert gas comprises heating at a rate of 1 to 50 degrees Celsius per minute, or 2 to 20 degrees Celsius per minute.

9. The process of any of the preceding claims, wherein each of the first temperature and the second temperature is within a range of 700 to 950 °C, or 725 to 900 °C.

10. The process of any of the preceding claims, wherein the gas comprising oxygen comprises 5 to 100 weight percent oxygen.

11. The process of any of the preceding claims, wherein the gas comprising oxygen comprises air.

12. The process of any of the preceding claims, wherein the inert gas comprises nitrogen, helium, argon, or a combination thereof.

13. The process of any of the preceding claims, wherein the dehydrogenation catalyst comprises:
gallium oxide;
cerium oxide;
a Group 1 metal oxide, and
a Group 8-11 metal oxide; and
a support.

14. A process of dehydrogenating an alkane, the process comprising:
heating a dehydrogenation catalyst to a first temperature in the presence of a gas comprising oxygen;
after heating the dehydrogenation catalyst to the first temperature in the presence of the gas comprising oxygen, heating the dehydrogenation catalyst to a second temperature in the presence of an inert gas;
repeating heating the dehydrogenation catalyst to the first temperature in the presence of the gas comprising oxygen and thereafter heating the dehydrogenation catalyst to the second temperature in the presence of the inert gas for 1 to 50 hours, or 2 to 20 hours; and
after the repeating, contacting the dehydrogenation catalyst with a feed comprising the alkane at a third temperature to produce a product stream comprising an alkene,
wherein the third temperature is less than the first temperature, less than the second temperature, and within a range of 500 to 700 °C, or 550 °C to 675 °C.

15. A process of dehydrogenating an alkane, the process comprising:
heating a dehydrogenation catalyst comprising gallium oxide, cerium oxide, a Group 1 metal oxide, and a Group 8-11 metal oxide on a support to a first temperature within a range of greater than 700 to 950 °C, or 725 to 900 °C, in the presence of a gas comprising oxygen;
after heating the dehydrogenation catalyst to the first temperature in the presence of the gas comprising oxygen, heating the dehydrogenation catalyst to a second temperature within a range of greater than 700 to 950 °C, or 725 to 900 °C, in the presence of an inert gas;
repeating heating the dehydrogenation catalyst to the first temperature in the presence of the gas comprising oxygen and thereafter heating the dehydrogenation catalyst to the second temperature in the presence of the inert gas for 1 to 50 hours, or 2 to 20 hours; and
after the repeating, contacting the dehydrogenation catalyst with a feed comprising the alkane at a third temperature within a range of 500 to less than 700 °C, or 550 °C to 675 °C, to produce a product stream comprising an alkene.
